(19) Europäisches Patentamt
European Patent Office
Office européen des brevets

(11) Publication number: **0 011 372**
**B1**

(12) **EUROPEAN PATENT SPECIFICATION**

(45) Date of publication of patent specification: **09.02.83**

(21) Application number: **79302169.2**

(22) Date of filing: **10.10.79**

(51) Int. Cl.[3]: **C 07 C 93/26,** C 07 C 93/06, A 61 K 31/215

(54) **1-Acyloxyphenyl-1,2-diphenylalkene derivatives, processes for their manufacture and a pharmaceutical composition containing these derivatives.**

(30) Priority: **07.11.78 GB 4343078**

(43) Date of publication of application: **28.05.80 Bulletin 80/11**

(45) Publication of the grant of the patent: **09.02.83 Bulletin 83/6**

(84) Designated Contracting States: **AT BE CH DE FR GB IT LU NL SE**

(56) References cited:
**AT - B - 345 804**
**DE - A - 1 493 855**
**DE - A - 2 807 599**
**GB - A - 945 864**
**US - A - 2 914 564**
**US - A - 3 237 200**

(73) Proprietor: **IMPERIAL CHEMICAL INDUSTRIES PLC**
**Imperial Chemical House Millbank**
**London SW1P 3JF (GB)**

(72) Inventor: **Richardson, Dora Nellie**
**3 Silverdale Road**
**Gatley, Cheshire (GB)**

(74) Representative: **Slatcher, Reginald Peter et al,**
***Imperial Chemical Industries PLC Legal* Department: Patents Thames House North Millbank**
**London SW1P 4QG (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

EP 0 011 372 B1

1-Acyloxyphenyl-1,2-diphenylalkene derivatives, processes for their manufacture and a pharmaceutical composition containing these derivatives

This invention relates to triphenylalkene derivatives which possess anti-oestrogenic activity. According to the invention there is provided an alkene derivative of the formula:—

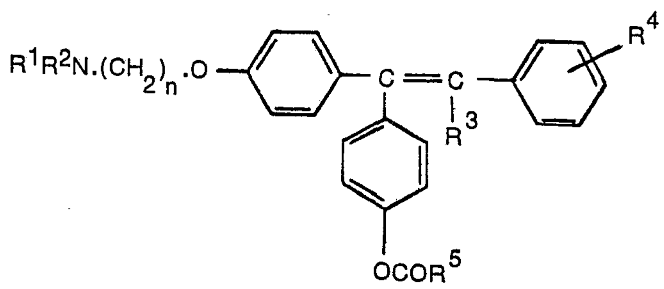

(I)

wherein either $R^1$ is a hydrogen atom or a lower alkyl radical and $R^2$ is a lower alkyl radical, or $R^1$ and $R^2$ are joined together with the adjacent nitrogen atom to form a heterocyclic radical, n is 2, 3, 4, 5 or 6; $R^3$ is a halogen atom or a lower alkyl radical; $R^4$ is a hydrogen or halogen atom, or a hydroxy or lower alkyl or lower alkoxy radical, or is the buta-1,3-dienyl radical such that together with the benzene ring it forms a naphthyl radical, or $R^4$ has the formula $—OCOR^5$; and $R^5$ is an alkyl radical of 1 to 10 carbon atoms or an alkoxyalkyl radical of 2 to 10 carbon atoms, or $R^5$ has the formula

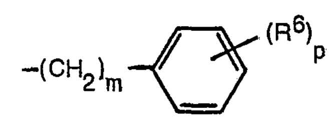

wherein m is 0, 1, 2, 3 or 4, wherein $R^6$ is a hydrogen or halogen atom or a cyano, lower alkyl or lower alkoxy radical and wherein p is 1, 2 or 3; or $R^5$ has the formula $—NHR^7$, wherein $R^7$ is an alkyl radical of 1 to 10 carbon atoms or $R^7$ has the formula

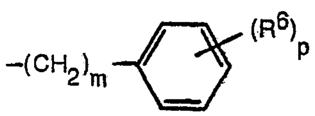

wherein m, p and $R^6$ have the meanings stated above; or a pharmaceutically-acceptable acid-addition salt thereof.

It is clear that a compound of the invention can exist in the form of a *cis* or a *trans* isomer in which the designation *cis* or *trans* refers to the relative positions of the acyloxyphenyl radical, and the phenyl radical bearing the group $R^4$, about the double bond. The *cis* and *trans* isomers may be distinguished by the magnetic resonance signals of the protons in the $—OCH_2—$ group of the $—O(CH_2)_nNR^1R^2$ side-chain, the signals of these protons in the *cis* isomers occurring at lower field than of those in the corresponding *trans* isomers. This invention includes both *cis* and *trans* isomers and mixtures thereof which possess the above properties, it being a matter of common general knowledge how to separate *cis* and *trans* isomers and how to determine their anti-oestrogenic and oestrogenic activity.

$R^1$, $R^2$, $R^3$, $R^4$ or $R^6$ when it is a lower alkyl radical is an alkyl radical of 1—4 carbon atoms, for example a methyl, ethyl, n-propyl, isopropyl or a butyl radical.

$R^1$ and $R^2$ when they are joined together with the adjacent nitrogen atom to form a heterocyclic radical form a 5- or 6- membered nitrogen-containing heterocyclic radical optionally including an oxygen or sulphur atom as a second hetero-atom, for example a pyrrolidino, piperidino or morpholino radical.

A particularly suitable value for $R^3$, $R^4$ or $R^6$ when it is a halogen atom is, for example, a fluorine, chlorine or bromine atom.

$R^4$ or $R^6$ when it is a lower alkoxy radical is an alkoxy radical of 1—4 carbon atoms, for example a methoxy or ethoxy radical.

A particularly suitable value for $R^5$ when it is an alkyl or alkoxyalkyl radical is, for example, the methyl, ethyl, isopropyl, hexyl, decyl or methoxymethyl radical.

A particularly suitable salt is, for example a hydrochloride, sulphate, phosphate, acetate, tartrate or citrate.

A preferred compound of the invention has formula I given above wherein $R^1$ and $R^2$ are both the same lower alkyl radical, preferably the methyl or ethyl radical and especially the methyl radical, wherein $R^3$ is a lower alkyl radical, preferably the methyl, ethyl or n-propyl radical and especially the ethyl radical, wherein $R^4$ is hydrogen or is a halogen atom or a lower alkyl or lower alkoxy radical, preferably the fluorine, chlorine or bromine atom or the methyl, ethyl or methoxy radical, and especially such a radical in the 4-position, wherein $R^5$ is the methyl, ethyl, isopropyl, t-butyl, methoxymethyl, phenyl, 2-tolyl, 4-tolyl, 2-ethylphenyl, 2-chlorophenyl, 3-chlorophenyl, 4-chlorophenyl, 4-bromophenyl, 4-methoxyphenyl, 4-cyanophenyl, benzyl, methylamino, propylamino or anilino radical, and wherein n is 2, 3, 4 or 5, preferably 2, or is a pharmaceutically-acceptable acid-addition salt thereof. In general, *trans*-isomers are more active than the corresponding *cis*-isomers.

Particular compounds of the invention are set out in the Examples, and preferred compounds are 1 - (*p* - *β* - dimethylaminoethoxyphenyl) - *trans* - 1 - *p* - acetoxyphenyl - 2 - *p* - tolylbut - 1 - ene, 1 - (*p* - *β* - dimethylaminoethoxyphenyl) - *trans* - 1 - *p* - acetoxyphenyl - 2 - phenylbut - 1 - ene, 1 - (*p* - *β* - dimethylaminoethoxyphenyl) - *trans* - 1 - *p* - benzoyloxyphenyl - 2 - phenylbut - 1 - ene, 1 - (*p* - *β* - dimethylaminoethoxyphenyl) - *trans* - 1 - *p* - propionyloxyphenyl - 2 - phenylbut - 1 - ene, 1 - (*p* - *β* - dimethylaminoethoxyphenyl) - *trans* - 1 - *p* - acetoxyphenyl - 2 - *p* - ethylphenylbut - 1 - ene and 1 - (*p* - *β* - dimethylaminoethoxyphenyl) - *trans* - 1 - *p* - benzoyloxyphenyl - 2 - *p* - tolylbut - 1 - ene.

A compound of formula I may be obtained by the processes which are applicable to the manufacture of analogous compounds. Thus, for example, an alkene derivative of the formula:—

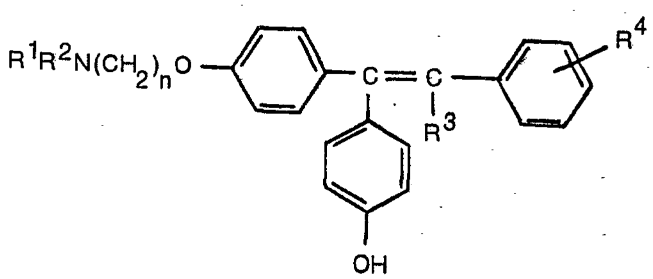

(II)

wherein $R^1$, $R^2$, $R^3$, $R^4$ and n have the meanings stated above (which alkene derivatives are mostly described in European Specification No. 0,002,097 or are otherwise known compounds, or may be obtained by analogous methods to those known or described in said Specification) may be acylated with an acylating derivative obtained from an acid of the formula $R^5COOH$, wherein $R^5$ has the meaning stated above, or, when $R^5$ has the formula —$NHR^7$, may be reacted with an isocyanate of the formula $R^7NCO$.

A suitable acylating derivative is, for example, an acyl chloride or an acid anhydride.

Alternatively, an alkanol of the formula:—

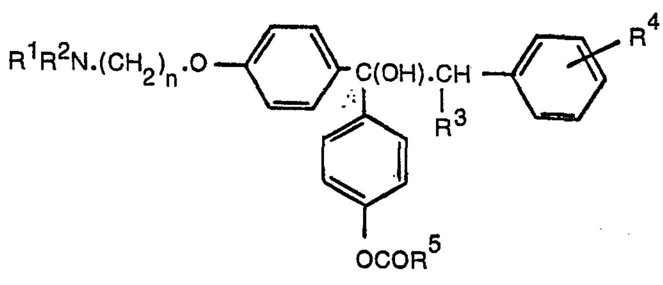

(III)

wherein $R^1$, $R^2$, $R^3$, $R^4$, $R^5$ and n have the meanings stated above, may be dehydrated with an acid, for example hydrochloric acid, conveniently in a solvent, for example ethanol, at a temperature of from 20°C. to 80°C.

The alkene derivative so obtained may be in the form of a mixture of the *cis* and *trans* isomers. If desired, the individual isomers may be obtained by fractional crystallisation of the mixture, or by chromatography.

The starting material of formula III may be obtained by the acylation with an acylating derivative, for example an acyl chloride or acid anhydride, obtained from an acid of the formula $R^5COOH$, wherein $R^5$ has the meaning stated above, of the corresponding hydroxy compound (which hydroxy compounds are mostly described in European Specification No. 0,002,097, or are known compounds, or may be obtained by analogous methods to those known or described in said Specification).

An alkene derivative of the invention in free base form may be converted into an acid-addition said thereof by reaction with an acid.

The anti-oestrogenic activity of a compound of formula I has been demonstrated by its effect in preventing implantation of the fertilised ovum when administered orally to rats on day 4 of pregnancy. In this test, each of the compounds showed substantial activity at a dose of 0.2 mg./kg. and a preferrred compound showed activity at a dose of 0.01 mg./kg. this compound therefore being substantially more active than tamoxifen. Anti-oestrogenic activity can also be demonstrated by inhibition of oestradiol-induced vaginal cornification in ovariectomised rats, and by inhibition of uterine growth when given concomitantly with an oestrogen to immature female rats.

The weak oestrogenic activity of a compound of formula I has been demonstrated by its effect in producing cornified vagina smears in spayed rats when administered orally once daily for 3 days. In this test, each of the compounds showed oestrogenic activity only at a dose substantially greater than that required to produce anti-oestrogenic effects.

A compound with the above pharmacological properties is of value in the treatment of the same conditions in which tamoxifen is beneficial, in particular, in the treatment of anovulatory infertility and in the treatment of breast tumours.

When used to produce an anti-oestrogenic effect in warm blooded animals, a typical daily dose is from 0.05 to 1 mg./kg. administered orally or by injection. In man this is equivalent to an oral dose of from 5—80 mg./day. In use, tamoxifen has been administered orally at doses of from 20—80 mg./day for the treatment of anovulatory infertility, and at doses from 10—40 mg./day for the treatment of breast tumors. A similar regime is appropriate for the administration of a compound of formula I, most conveniently in the form of a pharmaceutical composition.

According to a further feature of the invention, there is provided a pharmaceutical composition comprising a compound of formula I or a pharmaceutically acceptable acid-addition salt thereof, together with a pharmaceutically acceptable diluent or carrier.

The composition may be in a form suitable for oral or parenteral administration. A tablet or capsule is a particularly convenient form for oral administration, and such a composition may be made by conventional methods and contain conventional excipients. Thus a tablet could contain diluents, for example mannitol or maize starch, disintegrating agents, for example, alginic acid, binding agents, for example methylcellulose, and lubricating agents, for example magnesium stearate.

A composition for oral administration may conveniently contain from 5—50 mg. of a compound of formula I, preferably 5—20 mg.

The invention is illustrated but not limited by the following Examples:—

### Example 1

A mixture of 1-(*p*-β-dimethylaminoethoxyphenyl)-*trans*-1-*p*-hydroxyphenyl-2-phenylbut-1-ene (1.0 g.), acetic anhydride (25 ml.) and anhydrous sodium acetate (0.5 g.) is heated under reflux for 30 minutes, cooled and poured into ice-water. The mixture is made alkaline with aqueous ammonium hydroxide solution and extracted with ethyl acetate. The extract is dried, evaporated to dryness and the residue is crystallised from petroleum ether (b.p. 80—100°C). There is thus obtained 1-(*p*-β-dimethylaminoethoxyphenyl)-*trans*-1-*p*-acetoxyphenyl-2-phenylbut-1-ene, m.p. 108—110°C.

The process described above is repeated using the appropriate but-1-ene derivative and the appropriate acid anhydride, and there are thus obtained the compounds of Formula I (in all of which $R^1$ and $R^2$ are both methyl, n is 2 and $R^3$ is ethyl) described in the following table:—

| $R^4$ | $R^5$ | isomer | m.p.(°C.) |
|---|---|---|---|
| H | methyl | cis | 70—74 |
| H | ethyl | trans | 91—93 |
| 4-methyl | methyl | trans | 106—108 |
| 4-methyl | methyl | cis | 68—70 |
| 4-acetoxy | methyl | trans | 96—99 |
| 4-ethyl | methyl | trans | 74 |
| 4-ethyl | methyl | cis | 78 |

Example 2

Benzoyl chloride (0.3 ml.) is added to a cooled solution of 1-(*p*-β-dimethylaminoethoxyphenyl)-*trans*-1-*p*-hydroxyphenyl-2-phenylbut-1-ene (0.4 g.) in pyridine (3 ml.) and the mixture is kept at 0°C. for 17 hours and then poured into water. The mixture is extracted with ether and the extract is dried and evaporated to dryness. The residue is dissolved in petroleum ether (b.p. 80—100°C.) and the solution is allowed to evaporate slowly. The residue crystallises and consists of 1-(*p*-β-dimethylaminoethoxy-phenyl)-*trans*-1-*p*-benzoyloxyphenyl-2-phenylbut-1-ene, m.p. 96—98°C.

The process described above is repeated using the appropriate acyl chloride and the appropriate 1-*p*-dialkylaminoalkoxy-1-*p*-hydroxyphenyl-2-phenylalk-1-ene derivative as starting materials and there are thus obtained the compounds described in the following tables:—

*Table 1:* Compounds have Formula I wherein $R^1$ and $R^2$ are both methyl, n is 2, $R^3$ is ethyl and $R^4$ is hydrogen:—

| $R^5$ | Isomer | m.p.(°C.) |
|---|---|---|
| phenyl | cis | 140—142 |
| 2-tolyl | trans | 115—117 |
| 4-tolyl | trans | 108—110 |
| 4-tolyl | cis | 109—111 |
| 4-methoxyphenyl | trans | 117—118 |
| 4-methoxyphenyl | cis | 118 |
| 2-chlorophenyl | trans | 108—110 |
| 3-chlorophenyl | trans | 76—78 |
| 4-chlorophenyl | trans | 80—82 |
| 4-chlorophenyl | cis | 90—92 |
| 4-bromophenyl | trans | 94—96 |
| 4-bromophenyl | cis | 94 |
| 2,6-dichlorophenyl | trans | 135—137 |
| isopropyl | trans | 104—107 |

*Table 2:* Compounds have Formula I wherein n is 2 and $R^5$ is unsubstituted phenyl.

| $R^1$ | $R^2$ | $R^3$ | $R^4$ | Isomer | m.p.(°C.) |
|---|---|---|---|---|---|
| methyl | methyl | isopropyl | H | trans | 120—124 |
| methyl | methyl | ethyl | 2-methyl | trans | 125 |
| methyl | methyl | ethyl | 4-methyl | trans | 113—115 |
| methyl | methyl | ethyl | 4-ethyl | trans | 173 |
| methyl | methyl | ethyl | 4-ethyl | cis | 95 |
| methyl | methyl | ethyl | 3-chloro | trans | (oil) |
| ethyl | ethyl | ethyl | 4-methoxy | trans | 99—101 |

Example 3

A solution of 1-(*p*-β-dimethylaminoethoxyphenyl)-*trans*-1-*p*-hydroxyphenyl-2-phenylbut-1-ene (0.77 g.) in tetrahydrofuran (15 ml.) is added to a stirred suspension of sodium hydride (0.5 g.) in tetrahydrofuran (10 ml.) and the mixture is stirred at laboratory temperature for 30 minutes. The solution is removed by pipette from the excess of sodium hydride and to this solution is added a solution of 2-ethylbenzoyl chloride (0.45 g.) in tetrahydrofuran (3 ml.). The mixture is stirred at laboratory temperature for 15 minutes, diethyl ether (30 ml.) is added and the solution is washed with saturated aqueous sodium bicarbonate solution and then with water. The ethereal solution is dried and evaporated to dryness and the residue is crystallised from hexane. There is thus obtained 1-(*p*-β-dimethylaminoethoxyphenyl)-*trans*-1-(2-ethylbenzoyloxyphenyl)-2-phenylbut-1-ene, m.p. 77—78°C.

The process described above is repeated using the appropriate acid chloride, in place of 2-ethylbenzoyl chloride, and there are thus obtained the compounds of formula I (in all of which $R^1$ and $R^2$ are both methyl, n is 2, $R^3$ is ethyl and $R^4$ is hydrogen) described in the following table:—

| $R^5$ | isomer | m.p.(°C.) |
|---|---|---|
| 2-tolyl | cis | 95—98 |
| 2,6-dichlorophenyl | trans | 126—127 |
| 4-cyanophenyl | trans | 172—174 |
| methoxymethyl | trans | 81—83 |
| methoxymethyl | cis | 70—72 |
| benzyl | cis | 84—86 |
| benzyl | trans | 100—102 |
| t-butyl | trans | 118—119 |

Example 4

A solution of phenyl isocyanate (0.298 g.) in dry *N,N*-dimethylformamide (2 ml.) is added during 10 minutes to a stirred solution of 1-(*p*-β-dimethylaminoethoxyphenyl)-*trans*-1-*p*-hydroxyphenyl-2-phenylbut-1-ene (0.774 g.) and triethylamine (0.02 g.) in dry *N,N*-dimethylformamide (5 ml.) which is cooled to 0°C. The mixture is stirred for 15 minutes at 0°C. and then for 17 hours at laboratory temperature, diethyl ether (30 ml.) is added and the mixture is washed three times with water (20 ml. each time), dried and evaporated to dryness. The residue is crystallised from cyclohexane and the solid product dissolved in methylene chloride. The mixture is filtered, the filtrate is evaporated to dryness and the residue is stirred with cold methanol. The mixture is filtered and there is thus obtained as solid residue 1-(*p*-β-dimethylaminoethoxyphenyl)-*trans*-1-(*p*-*N*-phenylcarbamoyloxyphenyl)-2-phenylbut-1-ene, m.p. 145—146°C.

The process described above is repeated except that methyl or propyl isocyanate is used in place of phenyl isocyanate. There are thus obtained the compounds of formula I (in all of which $R^1$ and $R^2$ are both methyl, n is 2, $R^3$ is ethyl and $R^4$ is hydrogen) described in the following table:—

| $R^5$ | isomer | m.p.(°C.) |
|---|---|---|
| methylamino | trans | 130—131 |
| propylamino | trans | 98—100 |

**Claims for the Contracting States: BE CH DE FR GB IT LU NL SE**

1. An alkene derivative of the formula:—

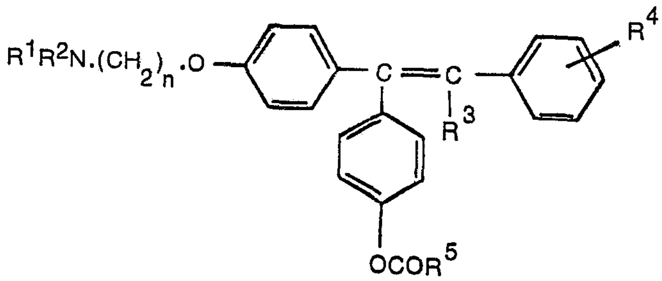

(I)

wherein either $R^1$ is a hydrogen atom or an alkyl radical of 1—4 carbon atoms and $R^2$ is an alkyl radical of 1—4 carbon atoms, or $R^1$ and $R^2$ are joined together with the adjacent nitrogen atom to form a 5- or 6-membered heterocyclic radical; n is 2, 3, 4, 5 or 6; $R^3$ is a halogen atom or an alkyl radical of 1—4 carbon atoms; $R^4$ is a hydrogen or halogen atom, or a hydroxy or alkyl or alkoxy radical each of 1—4 carbon atoms, or is the buta-1,3-dienyl radical such that together with the benzene ring it forms a naphthyl radical, or $R^4$ has the formula —$OCOR^5$; and $R^5$ is an alkyl radical of 1 to 10 carbon atoms or an alkoxyalkyl radical of 2 to 10 carbon atoms, or $R^5$ has the formula

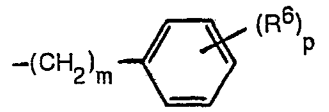

wherein m is 0, 1, 2, 3 or 4, wherein $R^6$ is a hydrogen or halogen atom or a cyano, or alkyl or alkoxy radical each of 1—4 carbon atoms, and wherein p is 1, 2 or 3; or $R^5$ has the formula —$NHR^7$, wherein $R^7$ is an alkyl radical of 1 to 10 carbon atoms or $R^7$ has the formula

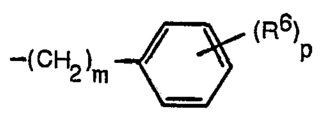

wherein m, p and $R^6$ have the meanings stated above, or a pharmaceutically-acceptable acid-addition salt thereof.

2. An alkene derivative as claimed in claim 1 wherein $R^1$ and $R^2$ are both methyl or both ethyl radicals, $R^3$ is the methyl, ethyl or propyl radical, $R^4$ is the hydrogen, fluorine, chlorine or bromine atom or the methyl, ethyl or methoxy radical, $R^5$ is the methyl, ethyl, isopropyl, t-butyl, methoxymethyl, phenyl, 2-tolyl, 4-tolyl, 2-ethylphenyl, 2-chlorophenyl, 3-chlorophenyl, 4-chlorophenyl, 4-bromophenyl, 4-methoxyphenyl, 4-cyanophenyl, benzyl, methylamino, propylamino or anilino radical and wherein n is 2, 3, 4 or 5, or a pharmaceutically acceptable acid-addition salt thereof.

3. An alkene derivative as claimed in claim 2 wherein $R^1$ and $R^2$ are both methyl radicals, $R^3$ is the ethyl radical, $R^4$ is the hydrogen atom or the chlorine atom or the methyl or ethyl radical in the 4-position, wherein $R^5$ is the methyl, ethyl, isopropyl or phenyl radical and wherein n is 2, or a pharmaceutically-acceptable acid-addition salt thereof.

4. The compound 1 - (*p* - *β* - dimethylaminoethoxyphenyl) - *trans* - 1 - *p* - acetoxyphenyl - 2 - *p* - tolylbut - 1 - ene, 1 - (*p* - *β* - dimethylaminoethoxyphenyl) - *trans* - 1 - *p* - acetoxyphenyl - 2 - phenyl - but - 1 - ene, 1 - (*p* - *β* - dimethylaminoethoxyphenyl) - *trans* - 1 - *p* - benzoyloxyphenyl - 2 - phenylbut-1 - ene or 1 - (*p* - *β* - dimethylaminoethoxyphenyl) - *trans* - 1 - *p* - propionyloxyphenyl - 2 - phenyl - but - 1 - ene.

5. The compound 1-(*p*-*β*-dimethylaminoethoxyphenyl)-*trans*-1-*p*-acetoxyphenyl-2-*p*-ethylphenylbut-1-ene or 1-(*p*-*β*-dimethylaminoethoxyphenyl)-*trans*-1-*p*-benzoyloxyphenyl-2-*p*-tolylbut-1-ene.

6. A process for the manufacture of an alkene derivative, claimed in any of claims 1 to 5, or a pharmaceutically-acceptable acid-addition salt thereof, which comprises either

(a) the acylation of an alkene derivative of the formula:—

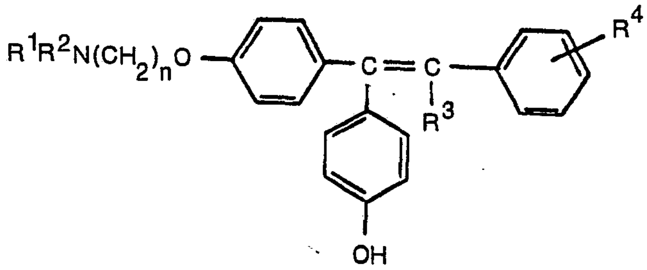

(II)

wherein $R^1$, $R^2$, $R^3$, $R^4$ and n have the meanings stated in claim 1 with an acylating derivative obtained from an acid of the formula $R^5COOH$, wherein $R^5$ has the meaning stated in claim 1, or, when $R^5$ has the formula —$NHR^7$, the reaction of said alkene derivative with an isocyanate of the formula $R^7NCO$; or

(b) the dehydration of an alkanol of the formula:—

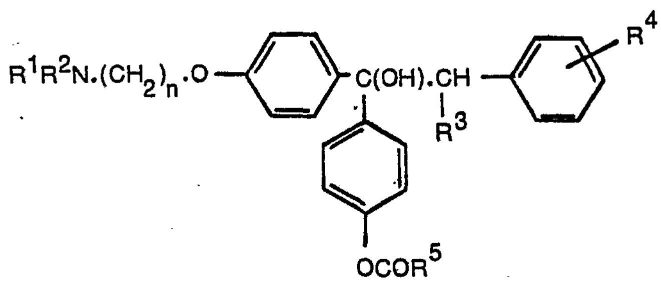

(III)

wherein $R^1$, $R^2$, $R^3$, $R^4$, $R^5$ and n have the meanings stated above, with an acid whereafter the alkene derivative may be separated into its *cis*- and *trans*-isomers; and whereafter an alkene derivative in free base form may be converted into an acid-addition salt thereof by reaction with an acid.

7. A pharmaceutical composition which comprises an alkene derivative or a pharmaceutically-acceptable acid-addition salt thereof claimed in any of claims 1 to 5, together with a pharmaceutically-acceptable diluent or carrier.

**Claims for the Contracting State: AT**

1. A process for the manufacture of an alkene derivative of the formula:—

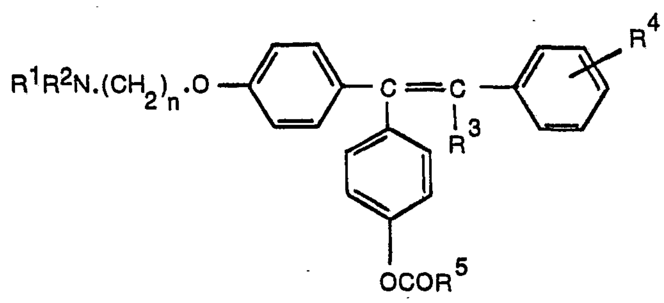

(I)

wherein either $R^1$ is a hydrogen atom or an alkyl radical of 1—4 carbon atoms and $R^2$ is an alkyl radical of 1—4 carbon atoms, or $R^1$ and $R^2$ are joined together with the adjacent nitrogen atom to form a 5- or 6-membered heterocyclic radical; n is 2, 3, 4, 5 or 6; $R^3$ is a halogen atom or an alkyl radical of 1—4 carbon atoms; $R^4$ is a hydrogen or halogen atom, or a hydroxy or alkyl or alkoxy radical each of 1—4 carbon atoms, or is the buta-1,3-dienyl radical such that together with the benzene ring it forms a naphthyl radical, or $R^4$ has the formula —$OCOR^5$; and $R^5$ is an alkyl radical of 1 to 10 carbon atoms or an alkoxyalkyl radical of 2 to 10 carbon atoms, or $R^5$ has the formula

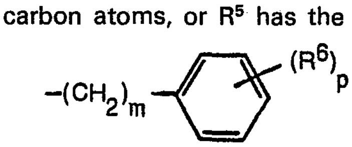

wherein m is 0, 1, 2, 3 or 4, wherein $R^6$ is a hydrogen or halogen atom or a cyano, or alkyl or alkoxy radical each of 1—4 carbon atoms and wherein p is 1, 2 or 3 or $R^5$ has the formula —$NHR^7$, wherein $R^7$ is an alkyl radical of 1 to 10 carbon atoms or $R^7$ has the formula

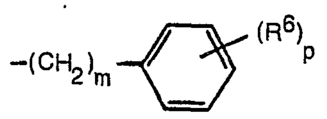

wherein m, p and $R^6$ have the meanings stated above, or a pharmaceutically-acceptable acid-addition salt thereof, characterised by either

(a) the acylation of an alkene derivative of the formula:—

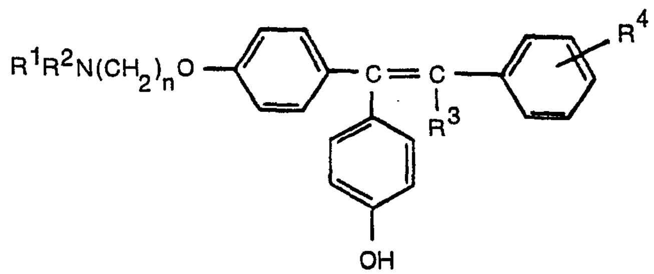

(II)

wherein $R^1$, $R^2$, $R^3$, $R^4$ and n have the meanings stated above with an acylating derivative obtained from an acid of the formula $R^5COOH$, wherein $R^5$ has the meaning stated above, or, when $R^5$ has the formula —$NHR^7$, the reaction of said alkene derivative with an isocyanate of the formula $R^7NCO$; or

(b) the dehydration of an alkanol of the formula:—

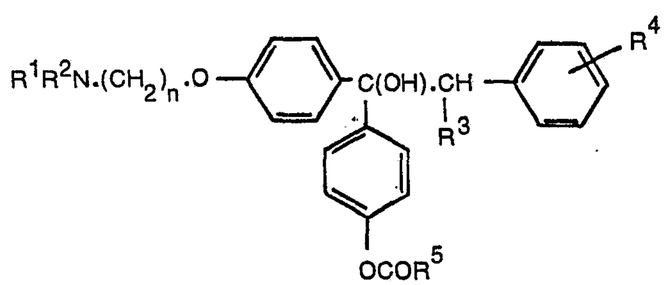

(III)

wherein $R^1$, $R^2$, $R^3$, $R^4$, $R^5$ and n have the meanings stated above, with an acid, whereafter the alkene derivative may be separated into its *cis*- and *trans*-isomers; and whereafter an alkene derivative in free base form may be converted into an acid-addition salt thereof by reaction with an acid.

2. A process for the manufacture of an alkene derivative of the formula:—

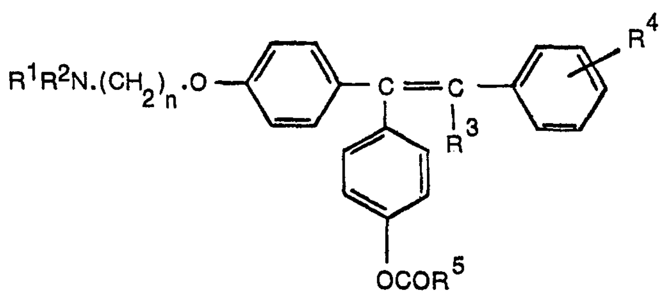

(I)

wherein $R^1$ and $R^2$ are both the same alkyl radical of 1—4 carbon atoms, wherein $R^3$ is an alkyl radical of 1—4 carbon atoms, wherein $R^4$ is hydrogen or is a halogen atom or an alkyl radical of 1—4 carbon atoms, wherein $R^5$ is methyl, ethyl, isopropyl or phenyl and wherein n is 2, 3, 4 or 5, or a pharmaceutically-acceptable acid-addition salt thereof, characterised by the acylation of an alkene derivative of the formula:—

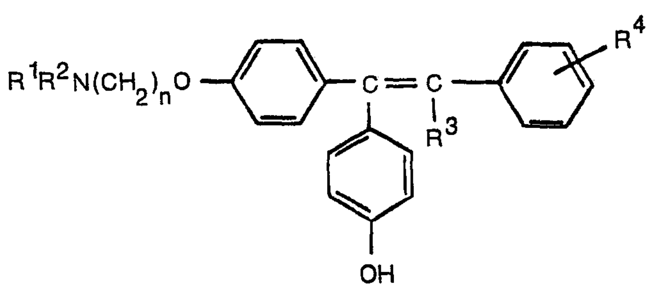

(II)

wherein $R^1$, $R^2$, $R^3$, $R^4$ and n have the meanings stated above, with an acylating derivative obtained from an acid of the formula $R^5COOH$, wherein $R^5$ has the meaning stated above, whereafter the alkene derivative may be separated into its *cis*- and *trans*-isomers.

**Patentansprüche für die Vertragsstaaten: BE CH DE FR GB IT LU NL SE**

1. Alkenderivat der Formel

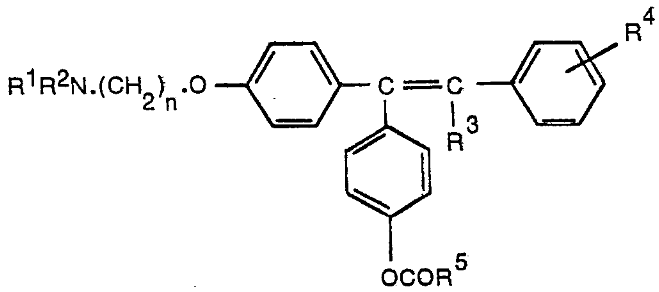

(I)

worin entweder $R^1$ für ein Wasserstoffatom oder ein Alkylradikal mit 1 bis 4 Kohlenstoffatomen steht und $R^2$ für ein Alkylradikal mit 1 bis 4 Kohlenstoffatomen steht oder $R^1$ und $R^2$ miteinander verbunden sind und mit dem benachbarten Stickstoffatom ein 5- oder 6-gliedriges heterocyclisches Radikal bilden; n für 2, 3, 4, 5 oder 6 steht; $R^3$ für ein Halogenatom oder ein Alkylradikal mit 1 bis 4 Kohlenstoffatomen steht; $R^4$ für ein Wasserstoff- oder Halogenatom oder ein Hydroxy- oder Alkyl- oder Alkoxyradikal mit jeweils 1 bis 4 Kohlenstoffatomen oder das Buta-1,3-dienylradikal, welches gemeinsam mit dem Benzolring ein Naphthylradikal bildet, steht oder $R^4$ die Formel —$OCOR^5$ aufweist; und $R^5$ für ein Alkylradikal mit 1 bis 10 Kohlenstoffatomen oder ein Alkoxyalkylradikal mit 2 bis 10 Kohlenstoffatomen steht oder $R^5$ die Formel

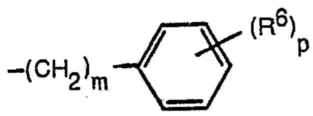

aufweist, wobei m für 0, 1, 2, 3, oder 4 steht, $R^6$ für ein Wasserstoff- oder Halogenatom oder ein Cyano- oder ein Alkyloder Alkoxyradikal mit jeweils 1 bis 4 Kohlenstoffatomen steht und p für 1, 2, oder 3 steht, oder $R^5$ die Formel —$NHR^7$ aufweist, worin $R^7$ für ein Alkylradikal mit 1 bis 10 Kohlenstoffatomen steht, oder $R^7$ die Formel

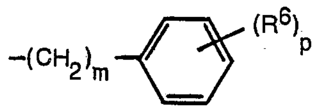

aufweist, wobei m, p und $R^6$ die oben angegebenen Bedeutungen besitzen; oder ein pharmazeutisch zulässiges Säureadditionssalz davon.

2. Alkenderivat nach Anspruch 1, worin $R^1$ und $R^2$ beide für Methyl- oder beide für Äthylradikale stehen, $R^3$ für das Methyl-, Äthyl- oder Propylradikal steht, $R^4$ für das Wasserstoff-, Fluor-, Chlor- oder Bromatom oder das Methyl-, Äthyl-, oder Methoxyradikal steht $R^5$ für das Methyl-, Äthyl-, Isopropyl-, t-Butyl-, Methoxymethyl-, Phenyl-, 2-Tolyl-, 4-Tolyl-, 2-Äthylphenyl-, 2-Chlorophenyl-, 3-Chlorophenyl-, 4-Chlorophenyl-, 4-Bromophenyl-, 4-Methoxyphenyl-, 4-Cyanophenyl-, Benzyl-, Methylamino-, Propylamino- oder Anilinoradikal steht und n für 2, 3, 4 oder 5 steht, oder ein pharmazeutisch zulässiges Säureadditionssalz davon.

3. Alkenderivat nach Anspruch 2, worin $R^1$ und $R^2$ beide für Methylradikale stehen, $R^3$ für das Äthylradikal steht, $R^4$ für das Wasserstoffatom oder das Chloratom oder das Methyl- oder Äthylradikal in der 4-Stellung steht, $R^5$ für das Methyl-, Äthyl-, Isopropyl- oder Phenylradikal steht und n für 2 steht, oder ein pharmazeutisch zulässiges Säureadditionssalz davon.

4. Die Verbindung 1-(p-β-Dimethylaminoäthoxyphenyl)-trans-1-p-acetoxyphenyl-2-p-tolylbut-1-en, 1-(p-β-Dimethylaminoäthoxyphenyl)-trans-1-p-acetoxyphenyl-2-phenylbut-1-en, 1-(p-β-Dimethylaminoäthoxyphenyl)-trans-1-p-benzoyloxyphenyl-2-phenylbut-1-en oder 1-(p-β-Dimethylaminoäthoxyphenyl)-trans-1-p-propionyloxyphenyl-2-phenylbut-1-en.

5. Die Verbindung 1-(p-β-Dimethylaminoäthoxyphenyl)-trans-1-p-acetoxyphenyl-2-p-äthylphenylbut-1-en oder 1-(p-β-Dimethylaminoäthoxyphenyl)-trans-1-p-benzoyloxyphenyl-2-p-tolylbut-1-en.

6. Verfahren zur Herstellung eines Alkenderivats nach einem der Ansprüche 1 bis 5 oder eines pharmazeutisch zulässigen Säureadditionssalzes davon, dadurch gekennzeichnet, daß entweder

(a) ein Alkenderivat der Formel

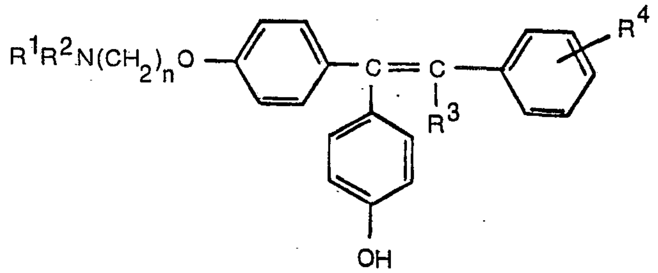

(II)

worin $R^1$, $R^2$, $R^3$, $R^4$ und n die in Anspruch 1 angegebenen Bedeutungen besitzen, mit einem acylierenden Derivat acyliert wird, das sich von einer Säure der Formel $R^5COOH$ ableitet, worin $R^5$ die in Anspruch 1 angegebene Bedeutung besitzt, oder, wenn $R^5$ die Formel $—NHR^7$ besitzt, dieses Alkenderivat mit einem Isocyanat der Formel $R^7NCO$ umgesetzt wird; oder

(b) ein Alkanol der Formel

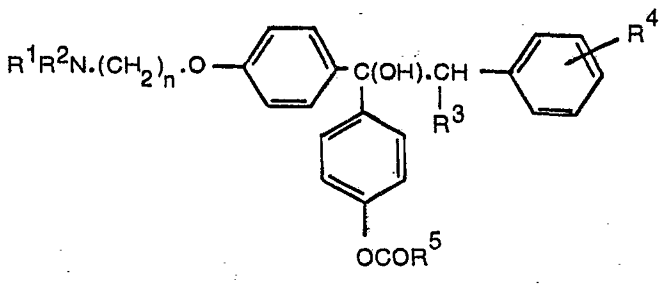

(III)

worin $R^1$, $R^2$, $R^3$, $R^4$, $R^5$ und n die oben angegebenen Bedeutungen besitzen, mit einer Säure dehydratisiert wird;

worauf das Alkenderivat in cis- und trans-Isomere getrennt werden kann; und

worauf ein Alkenderivat in der freien Basenform durch Umsetzung mit einer Säure in ein Säureadditionssalz umgewandelt werden kann.

7. Pharmazeutische Zusammensetzung, welche ein Alkenderivat oder ein pharmazeutisch zulässiges Säureadditionssalz davon nach einem der Ansprüche 1 bis 5 zusammen mit einem pharmazeutisch zulässigen Verdünnungsmittel oder Trägermittel enthält.

**Patentansprüche für den Vertragsstaat: AT**

1. Verfahren zur Herstellung eines Alkenderivats der Formel

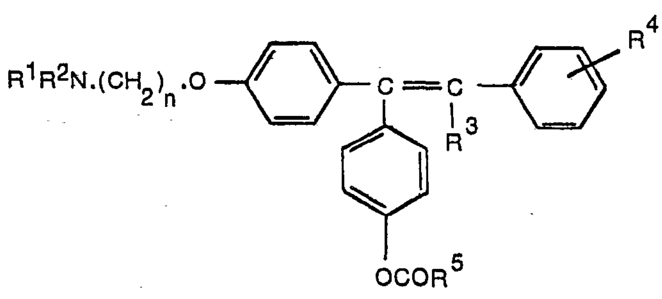

(I)

worin entweder $R^1$ für ein Wasserstoffatom oder ein Alkylradikal mit 1 bis 4 Kohlenstoffatomen steht und $R^2$ für ein Alkylradikal mit 1 bis 4 Kohlenstoffatomen steht oder $R^1$ und $R^2$ miteinander verbunden sind und mit dem benachbarten Stickstoffatom ein 5- oder 6-gliedriges heterocyclisches Radikal bilden; n für 2, 3, 4, 5 oder 6 steht; $R^3$ für ein Halogenatom oder ein Alkylradikal mit 1 bis 4 Kohlenstoffatomen steht; $R^4$ für ein Wasserstoff- oder Halogenatom oder ein Hydroxy- oder Alkyl- oder Alkoxyradikal mit jeweils 1 bis 4 Kohlenstoffatomen oder das Buta-1,3-dienylradikal, welches gemeinsam mit dem Benzolring ein Naphthylradikal bildet, steht oder $R^4$ die Formel $—OCOR^5$ aufweist; und $R^5$ für ein Alkylradikal mit 1 bis 10 Kohlenstoffatomen oder ein Alkoxyalkylradikal mit 2 bis 10 Kohlenstoffatomen steht oder $R^5$ die Formel

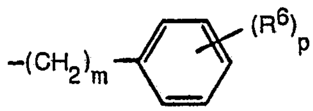

aufweist, wobei m für 0, 1, 2, 3 oder 4 steht, $R^6$ für ein Wasserstoff- oder Halogenatom oder ein Cyano- oder ein Alkyloder Alkoxyradikal mit jeweils 1 bis 4 Kohlenstoffatomen steht und p für 1, 2, oder 3 steht, oder $R^5$ die Formel —$NHR^7$ aufweist, worin $R^7$ für ein Alkylradikal mit 1 bis 10 Kohlenstoffatomen steht oder $R^7$ die Formel

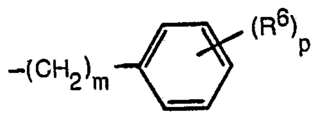

aufweist, wobei m, p und $R^6$ die oben angegebenen Bedeutungen besitzen; oder eines pharmazeutisch zulässigen Säureadditionssalzes davon, dadurch gekennzeichnet, daß

a) entweder ein Alkenderivat der Formel

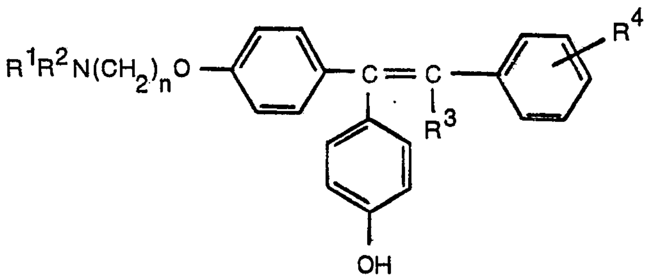

(II)

worin $R^1$, $R^2$, $R^3$, $R^4$ und n die oben angegebenen Bedeutungen besitzen, mit einem acylierenden Derivat acyliert wird, das sich von einer Säure der Formel $R^5COOH$ ableitet, worin $R^5$ die oben angegebene Bedeutung besitzt, oder, wenn $R^5$ die Formel —$NHR^7$ besitzt, dieses Alkenderivat mit einem Isocyanat der Formel $R^7NCO$ umgesetzt wird; oder

b) ein Alkanol der Formel

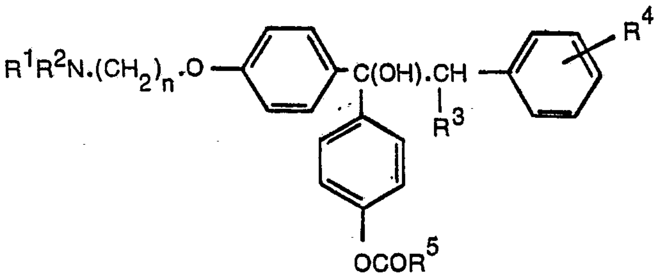

(III)

worin $R^1$, $R^2$, $R^3$, $R^4$, $R^5$ und n die oben angegebenen Bedeutungen besitzen, mit einer Säure dehydratisiert wird,

worauf das Alkenderivat in seine cis- und trans-Isomere getrennt werden kann; und

worauf ein Alkenderivat in der freien Basenform durch Umsetzung mit einer Säure in ein Säureadditionssalz überführt werden kann.

2. Verfahren zur Herstellung eines Alkenderivats der Formel

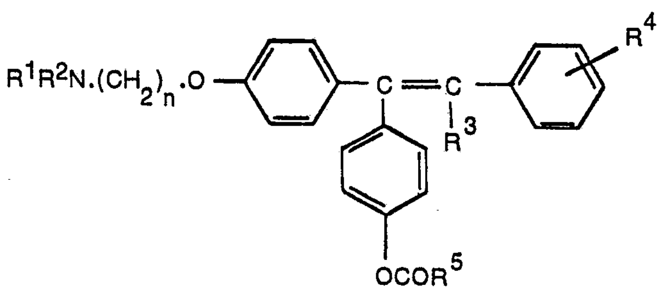

(I)

worin $R^1$ und $R^2$ jeweils für das gleiche Alkylradikal mit 1 bis 4 Kohlenstoffatomen stehen, $R^3$ für ein Alkylradikal mit 1 bis 4 Kohlenstoffatomen steht, $R^4$ für Wasserstoff oder ein Halogenatom oder ein Alkylradikal mit 1 bis 4 Kohlenstoffatomen steht, $R^5$ für Methyl, Äthyl, Isopropyl oder Phenyl steht und n für 2, 3, 4 oder 5 steht, oder eines pharmazeutisch zulässigen Säureadditionssalzes davon, dadurch gekennzeichnet, daß ein Alkenderivat der Formel

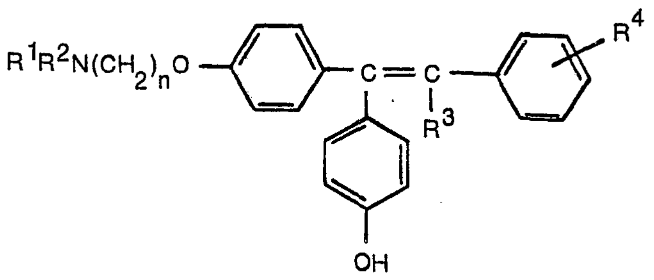

(II)

worin $R^1$, $R^2$, $R^3$, $R^4$ und n die oben angegebenen Bedeutungen besitzen, mit einem acylierenden Derivat, das sich von einer Säure der Formel $R^5COOH$ ableitet, worin $R^5$ die oben angegebene Bedeutung besitzt, acyliert wird,
worauf das Alkenderivat in seine cis- und trans-Isomere getrennt werden kann.

**Revendications pour les Etats contractants: BE CH DE FR GB IT LU NL SE**

1. Un dérivé alcénique de formule:

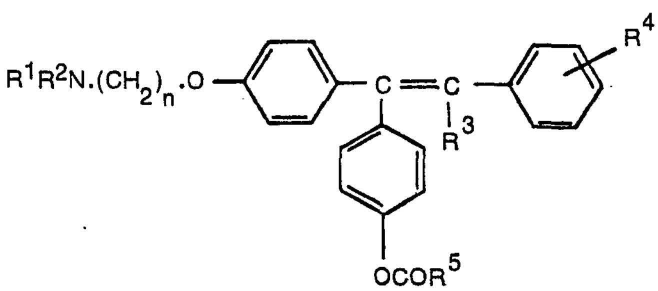

(I)

dans laquelle $R^1$ est un atome d'hydrogène ou un radical alkyle ayant 1 à 4 atomes de carbone et $R^2$ est un radical alkyle ayant 1 à 4 atomes de carbone, ou bien $R^1$ et $R^2$ forment conjointement avec l'atome adjacent d'azote un radical hétérocyclique pentagonal ou hexagonal, *n* est égal à 2, 3, 4, 5 ou 6; $R^3$ est un atome d'halogène ou un radical alkyle ayant 1 à 4 atomes de carbone; $R^4$ est un atome d'hydrogène ou d'halogène, ou un radical hydroxy ou alkyle ou alkoxy ayant chacun 1 à 4 atomes de carbone, ou représente le radical buta-1,3-diényle de manière qu'il forme conjointement avec le noyau benzénique un radical naphtyle, ou bien $R^4$ répond à la formule $—OCOR^5$; et $R^5$ est un radical alkyle ayant 1 à 10 atomes de carbone ou un radical alkoxyalkyle ayant 2 à 10 atomes de carbone, ou bien $R^5$ répond à la formule:

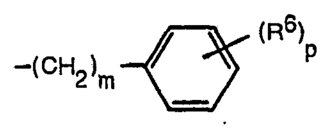

dans laquelle *m* est égal à 0, 1, 2, 3 ou 4, $R^6$ est un atome d'hydrogène ou d'halogène ou un radical cyano ou alkyle ou alkoxy ayant chacun 1 à 4 atomes de carbone et *p* est égal à 1, 2 ou 3; ou bien $R^5$ répond à la formule $—NHR^7$ dans laquelle $R^7$ est un radical alkyle ayant 1 à 10 atomes de carbone, ou bien $R^7$ répond à la formule:

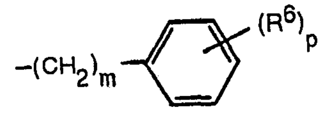

dans laquelle *m*, *p* et $R^6$ ont les définitions indiquées ci-dessus, ou un sel d'addition d'acide pharmaceutiquement acceptable de ce dérivé.

2. Un dérivé alcénique suivant la revendication 1, dans lequel $R^1$ et $R^2$ sont tous deux un radical méthyle ou tous deux un radical éthyle, $R^3$ est le radical méthyle, éthyle ou propyle, $R^4$ est un atome

d'hydrogène, de fluor, de chlore ou de brome ou le radical méthyle, éthyle ou méthoxy, $R^5$ est le radical méthyle, éthyle, isopropyle, tertio-butyle, méthoxyméthyle, phényle, 2-tolyle, 4-tolyle, 2-éthylphényle, 2-chlorophényle, 3-chlorophényle, 4-chlorophényle, 4-bromophényle, 4-méthoxyphényle, 4-cyanophényle, benzyle méthylamino, propylamino ou anilino et *n* est égal à 2, 3, 4 ou 5, ou un sel d'addition d'acide pharmaceutiquement acceptable de ce dérivé.

3. Un dérivé alcénique suivant la revendication 2, dans lequel $R^1$ et $R^2$ sont tous deux un radical méthyle, $R^3$ est le radical éthyle, $R^4$ est un atome d'hydrogène ou un atome de chlore ou le radical méthyle ou éthyle en position 4, $R^5$ est le radical méthyle, éthyle, isopropyle ou phényle et *n* est égal à 2, ou un sel d'addition d'acide pharmaceutiquement acceptable de ce dérivé.

4. Le 1-(*p*-β-diméthylaminoéthoxyphényl)-*trans*-1-p-acétoxyphényl-2-*p*-tolylbut-1-ène, le 1-(*p*-β-diméthylaminoéthoxyphényl)-*trans*-1-*p*-acétoxyphényl-2-phénylbut-1-ène, le 1-(*p*-β-diméthylaminoéthoxyphényl)-*trans*-1-*p*-benzoyloxyphényl-2-phénylbut-1-ène ou le 1-(*p*-β-diméthylaminoéthoxyphényl)-*trans*-1-*p*-propionyloxyphényl-2-phénylbut-1-ène.

5. Le 1-(*p*-β-diméthylaminoéthoxyphényl)-*trans*-1-*p*-acétoxyphényl-2-*p*-éthylphénylbut-1-ène ou le 1-(*p*-β-diméthylaminoéthoxyphényl)-*trans*-1-*p*-benzoyloxyphényl-2-*p*-tolylbut-1-ène.

6. Procédé de production d'un dérivé alcénique suivant l'une quelconque des revendications 1 à 5 ou d'un sel d'addition d'acide pharmaceutiquement acceptable de ce dérivé, qui comprend

(a) l'acylation d'un dérivé alcénique de formule:

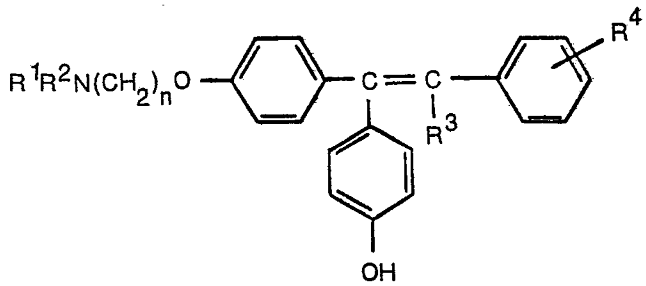

(II)

dans laquelle $R^1$, $R^2$, $R^3$, $R^4$ et *n* ont les définitions données dans la revendication 1, avec un dérivé acylant obtenu à partir d'un acide de formule $R^5COOH$, dans laquelle $R^5$ a la définition indiquée dans la revendication 1, ou bien, lorsque $R^5$ répond à la formule —$NHR^7$, la réaction dudit dérivé alcénique avec un isocyanate de formule $R^7NCO$; ou

(b) la déshydratation d'un alcanol de formule:

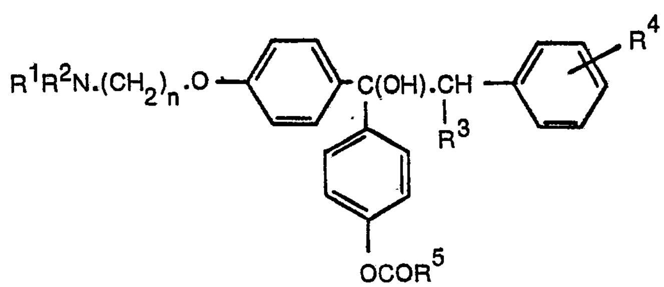

(III)

dans laquelle $R^1$, $R^2$, $R^3$, $R^4$, $R^5$ et *n* ont les définitions indiquées ci-dessus, avec un acide

après quoi le dérivé alcénique peut être divisé en ses isomères *cis* et *trans*; puis un dérivé alcénique sous la forme de la base libre peut être converti en un sel d'addition d'acide par réaction avec un acide.

7. Composition pharmaceutique, qui comprend un dérivé alcénique ou un sel d'addition d'acide pharmaceutiquement acceptable de ce dérivé suivant l'une quelconque des revendications 1 à 5, en association avec un diluant ou excipient pharmaceutiquement acceptable.

**Revendications pour l'Etat contractant: AT**

1. Procédé de préparation d'un dérivé alcénique de formule:

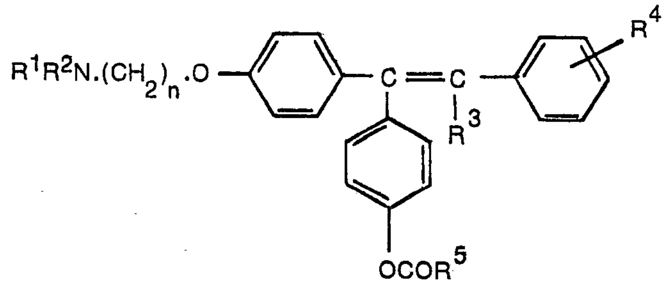

(I)

dans laquelle $R^1$ est un atome d'hydrogène ou un radical alkyle ayant 1 à 4 atomes de carbone et $R^2$ est un radical alkyle ayant 1 à 4 atomes de carbone, ou bien $R^1$ et $R^2$ forment conjointement avec l'atome adjacent d'azote un radical hétérocyclique pentagonal ou hexagonal; *n* est égal à 2, 3, 4, 5 ou 6; $R^3$ est un atome d'halogène ou un radical alkyle ayant 1 à 4 atomes de carbone; $R^4$ est un atome d'hydrogène ou d'halogène ou un radical hydroxy ou alkyle ou alkoxy ayant chacun 1 à 4 atomes de carbone, ou représente le radical buta-1,3-diényle, en sorte qu'il forme conjointement avec le noyau benzénique un radical naphtyle, ou bien $R^4$ répond à la formule —$OCOR^5$; et $R^5$ est un radical alkyle ayant 1 à 10 atomes de carbone ou un radical alkoxyalkyle ayant 2 à 10 atomes de carbone, ou bien $R^5$ répond à la formule:

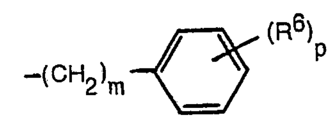

dans laquelle *m* est égal à 0, 1, 2, 3 ou 4, $R^6$ est un atome d'hydrogène ou d'halogène ou un radical cyano ou alkyle ou alkoxy ayant chacun 1 à 4 atomes de carbone et *p* est égal à 1, 2 ou 3, ou bien $R^5$ répond à la formule —$NHR^7$, dans laquelle $R^7$ est un radical alkyle ayant 1 à 10 atomes de carbone, ou bien $R^7$ répond à la formule:

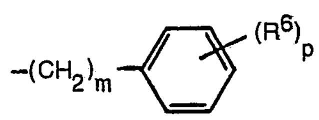

dans laquelle *m*, *p* et $R^6$ ont les définitions indiquées ci-dessus, ou d'un sel d'addition d'acide pharmaceutiquement acceptable de ce dérivé, caractérisé par

(a) l'acylation d'un dérivé alcénique de formule:

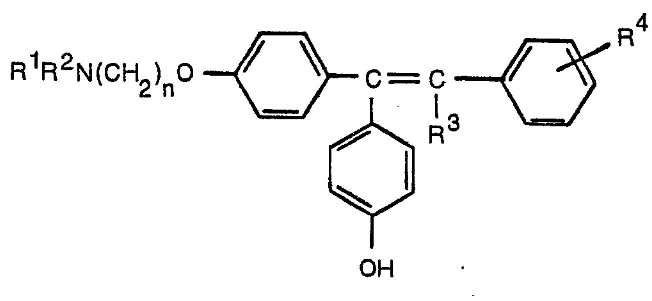

(II)

dans laquelle $R^1$, $R^2$, $R^3$, $R^4$ et *n* ont les définitions indiquées ci-dessus, avec un dérivé acylant obtenu à partir d'un acide de formule $R^5COOH$, dans laquelle $R^5$ a la définition donnée ci-dessus, ou bien lorsque $R^5$ répond à la formule —$NHR^7$, réaction dudit dérivé alcénique avec un isocyanate de formule $R^7NCO$; ou

(b) la déshydratation d'un alcanol de formule:

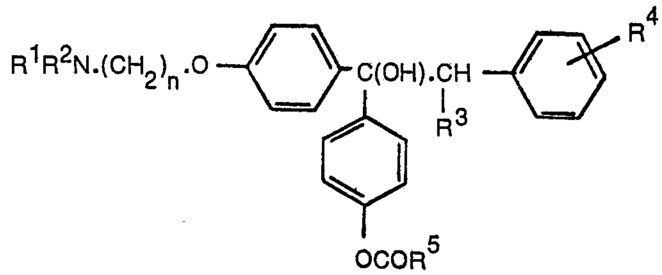

(III)

dans laquelle $R^1$, $R^2$, $R^3$, $R^4$, $R^5$ et *n* ont les définitions indiquées ci-dessus, avec un acide,

après quoi le dérivé alcénique peut être divisé en ses isomères *cis* et *trans*;

puis un dérivé alcénique sous la forme de la base libre peut être converti en un sel d'addition d'acide par réaction avec un acide.

2. Procédé de préparation d'un dérivé alcénique de formule:

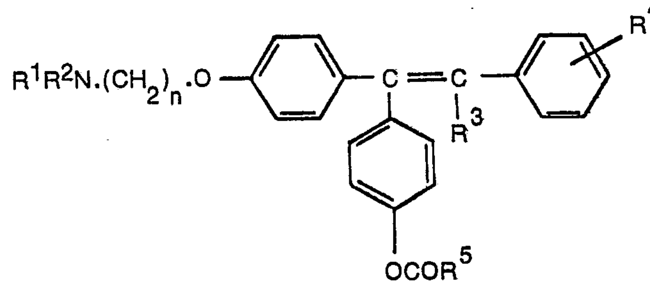

(I)

dans laquelle $R^1$ et $R^2$ représentent tous deux le même radical alkyle ayant 1 à 4 atomes de carbone, $R^3$ est un radical alkyle ayant 1 à 4 atomes de carbone, $R^4$ est un atome d'hydrogène ou un atome d'halogène ou un radical alkyle ayant 1 à 4 atomes de carbone, $R^5$ est un radical méthyle, éthyle, isopropyle ou phényle et *n* est égal à 2, 3, 4 ou 5, ou d'un sel d'addition d'acide pharmaceutiquement acceptable de ce dérivé, caractérisé par l'acylation d'un dérivé alcénique de formule:

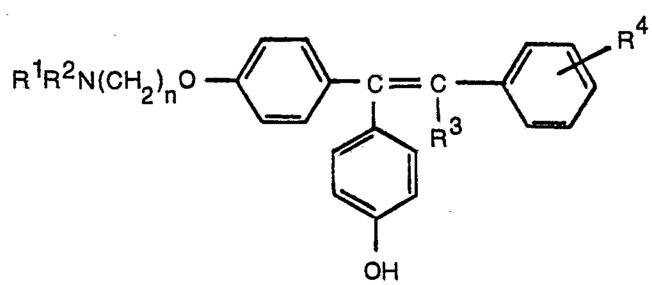

(II)

dans laquelle $R^1$, $R^2$, $R^3$, $R^4$ et *n* ont les définitions indiquées ci-dessus, avec un dérivé acylant obtenu à partir d'un acide de formule $R^5COOH$, dans laquelle $R^5$ a la définition indiquée ci-dessus,

après quoi le dérivé alcénique peut être divisé en ses isomères *cis* et *trans*.